# EUROPÄISCHE PATENTSCHRIFT

(11) **EP 1 677 787 B1**
(45) Veröffentlichungstag und Bekanntmachung des Hinweises auf die Patenterteilung: **12.12.2012**
(21) Anmeldenummer: 04790551.8
(22) Anmeldetag: 18.10.2004
(51) Int. Cl.: A61K 31/44, A61K 31/27, A61K 31/4453, A61P 25/02, A61P 25/06

(54) **KOMBINATIONEN AUS KALIUMKANAL ÖFFNERN UND NATRIUMKANALINHIBIT ODER NATRIUMKANALOREN BEEINFLUSSENDEN WIRKSTOFFEN ZUR BEHANDLUNG VON SCHMERZZUSTÄNDEN**
COMBINATIONS OF POTASSIUM CHANNEL OPENERS AND SODIUM CHANNEL INHIBITORS OR ACTIVE SUBSTANCES INFLUENCING SODIUM CHANNELS IN ORDER TO TREAT PAINFUL CONDITIONS
ASSOCIATIONS D'OUVREURS DES CANAUX POTASSIQUES ET D'INHIBITEURS DES CANAUX SODIQUES OU DE PRINCIPES ACTIFS INFLUEN ANT LES CANAUX SODIQUES UTILISEES POUR TRAITER DES ETATS DOULOUREUX

(30) Priorität: 23.10.2003 DE 10349729; 16.12.2003 DE 10359335
(43) Veröffentlichungstag der Anmeldung: 12.07.2006
(73) Patentinhaber: MEDA Pharma GmbH & Co. KG, 61352 Bad Homburg (DE)
(72) Erfinder: HERMANN, Robert, 78315 Radolfzell (DE); LOCHER, Mathias, 63549 Ronneburg (DE); SZELENYI, Istvan, 90571 Schwaig (DE); BRUNE, Kay, 91080 Marloffstein (DE)
(86) Internationale Anmeldenummer: PCT/EP2004/011718
(87) Internationale Veröffentlichungsnummer: WO 2005/039576

(56) Entgegenhaltungen:
- EP-A- 0 189 788
- WO-A-00/59487
- WO-A-00/59508
- DE-A1- 3 604 575
- KUO C C ET AL: "Inhibition of Na(+) current by diphenhydramine and other diphenyl compounds: molecular determinants of selective binding to the inactivated channels." MOLECULAR PHARMACOLOGY. JAN 2000, Bd. 57, Nr. 1, Januar 2000 (2000-01), Seiten 135-143, XP002316282 ISSN: 0026-895X
- DATABASE WPI Section Ch, Week 200037 Derwent Publications Ltd., London, GB; Class B03, AN 2000-426078 XP002316411 & JP 2000 143510 A (TAISHO PHARM CO LTD) 23. Mai 2000 (2000-05-23)
- BECK L ET AL: "KREUZSCHMERZEN IN DER GYNAEKOLOGISCHEN PRAXIS BACK PAIN IN GYNAECOLOGICAL PRACTICE" GYNAEKOLOGE, SPRINGER VERLAG, BERLIN, DE, Bd. 35, Nr. 5, 2002, Seiten 490-494, XP009041117 ISSN: 0017-5994
- SHAHNAZ C.A.: "The potassium Channel modulator flupirtine shifts the frequency response function of hyppocampal synapses to favour of LTD in ice", NEUROSCIENCE LETTERS, vol. 370, 2004, pages 186-190,
- MAYER-CHLOND G.: "Natriumkanal.Blokade durch Tolperidon", DEUTSCHE APOTHEKER ZEITUNG, vol. 142, no. 51-52, 2002,
- MERK: "Merk Index, 13th Ed.", 2001, MERCK * See potassium channel opener; pages THER-27 *

## Beschreibung

Die Erfindung bezieht sich auf ArzneimittelKombinationen aus dem Kaliumkanalöffne Flupirtin und Natriumkanalinhibitoren aus der Gruppe enthaltend Tolperison oder seine Analoga Eperison oder Silperison, oder deren pharmazeutisch verwendbare Salze zur Herstellung eines Arzneimittels zur Behandlung von Schmerzzuständen, die mit erhöhtem Muskeltonus einhergehen.

Eine Reihe von verschiedenen schmerzhaften Erkrankungen geht mit erhöhtem Skelettmuskeitonus einher In einigen Fällen wird die Schmerzentwicklung durch Entzündungen im Gelenk ausgelöst, und als Konsequenz entsteht eine schmerzhafte Körperhaltung, die oft von schmerzhaften Muskelspasmen begleitet ist. Die Therapie solcher Erkrankungen umfaßt z.B. Benzodiazepine, die jedoch ein deutliches Suchtpotenzial beinhalten und dadurch in ihrem Einsatz limitiert sind. Die Behandlung der grundlegenden Erkrankung z.B. der rheumatoiden Entzündung resultiert oft nicht in entsprechenden zufriedenstellenden therapeutischen Erfolgen. Deshalb ist die zusätzliche Gabe von Analgetika und/oder Skelettmuskelrelaxantien häufig indiziert.

Zentral wirkende Muskelrelaxantien werden in der klinischen Praxis eingesetzt, um unnormal erhöhten Muskeltonus in Patienten zu lindem, die an schmerzhaften Muskelspasmen und/oder Rigidität bei rheumatoiden Erkrankungen oder Spasmen im Zusammenhang mit neurologischen Erkrankungen leiden. Eine Reihe von entsprechenden Wirkstoffen befindet sich auf dem Markt, deren klinische Wirksamkeit jedoch oft fragwürdig ist oder durch unerwünschte Nebeneffekte limitiert ist.

Eine Klasse solcher Wirkstoffe sind die Na⁺-Kanal inhibierenden Substanzen. Zu dieser Klasse gehört beispielsweise die Substanz Diclofenac, die als Anlagetikum eingesetzt wird (Kuo et al. Mol. Pharmacology 2000; 57 Nr. 1: 135 - 143). Es gibt Hinweise darauf, dass diese in der Lage sind, einen erhöhten Muskeltonus zu lösen. Es konnte gezeigt werden, dass Propofol in klinisch relevanten Konzentrationen deutlich inhibitorisch auf die Natriumkanäle des Sarcolemma wirkt. Dieser Mechanismus könnte zu der Reduktion des Muskeltonus beitragen (Haeseler et al., Anesth Analg 2001; 92:1192-8). Es konnte ebenfalls gezeigt werden, dass die Inhibition der Na⁺-Kanäle die Inhibition der Neurotransmitterausschüttung an den präsynaptischen Enden bewirkt (Obrenovitch, Int Rev Neurobiol 1997;40:109-35). Der neuroprotektive Wirkstoff Riluzol ist ein Natriumkanalinhibitor und eine anti-excitotoxische Substanz, die zur Behandlung der amyotrophen Lateralsklerose eingesetzt wird. Kennel et al. (J Neurol Sci 2000; 180:55-61) konnten kürzlich zeigen, dass Riluzol den Beginn der Paralyse signifikant verzögert und den Fortschritt der funktionalen Parameter in Verbindung mit der Muskelstärke im Mausmodell der Motoneuronenkrankheit verlangsamt. Metilexin, eine antiarrhythmische und antimyotonische Substanz, blockiert die Natriumkanäle der Skelettmuskeln (Duranti et al., Eur J Med Chem 2000; 35:147-56) und löst die Übererregbarkeit der Skelettmuskeln im Mausmodell der vereerbbaren Myotonie (De Luca et al., J Pharmacol Exp Ther 1997; 282:93-100). Die wichtige Funktion der Natriumkanäle der Skelettmuskeln bei der Erhaltung des normalen Tonus wird durch die Tatsache, dass Mutationen im Gen für die α-Untereinheit des spannungsinduzierten Na⁺-Kanals (SCN4A) mit vererbter nicht dystropher Myotonie in Verbindung gebracht werden konnten. Interessanterweise löste sich die Myotonie dramatisch auf die Gabe der Na⁺-Kanal inhibierenden Substanz Flecai nid (Rosenfeld et al., Ann Neurol 1997; 42:811-4).

Tolperison ist ein zentral wirkendes Muskelrelaxans mit einer relativ guten klinischen Verträglichkeit. Bisher befassen sich relativ wenige Veröffentlichungen mit dem Wirkmechanismus Tolperison-ähnlicher Verbindungen. Tolperison unterdrückt die Weiterleitung des spinalen Segmentreflexes und reduziert wirksam die durch C Fasern induzierte Weiterleitung der afferenten Nerven sowohl *in vivo* als auch *in vitro* (Farkas et al., Neurobiology 1997; 5:57-58). Im Vergleich zu Lidocain, einem Lokalanästhetikum, wirkt die Substanz weniger blockierend auf die Weiterleitung der A Fasern. Seine charakteristischste Wirkung ist die starke Inhibition der mono- und polysynaptischen spinalen Reflexe (Farkas et al. Neurobiology 1997; 5:57-58, Kocsis et al., Acta Pharm Hung 2002;72(1):49-61, Okada et al., Jpn J Pharmacol 2001; 86:134-136). Ono et al. (J Pharmacobio Dynam 1984; 7:171-178) konnten zeigen, dass Tolperison eine Wirkung ähnlich einem Lokalanästhetikum ("Membranstabilisierend") sowohl in Motoneuronen als auch in primary afferents *in vivo,* als auch auf die peripheren Nerven bei Ratten *in vitro* zeigt. Der Effekt von Tolperison scheint ähnlich dem von Lidocain zu sein, von dem bekannt ist, dass es als Inhibitor von spannungsabhängigen Natriumkanälen wirkt (Strathmann 2002, www.ifap-index.de/bda/hausarzt/19-2002/64-83.pdf). Es konnte gezeigt werden, dass Tolperison, ähnlich wie Lidocain, die Tetrodotoxin (TTX) sensitiven und TTX resistenten Ströme blockiert und so eine inhibitorische Wirkung auf beide Arten spannungsabhängiger Natriumkanäle nahelegt (Bastigkeit, MMW-Forschr Med 2000; 142:50-51, Farkas et al., 2000, http://www.asso.univ-paris5.fr/ewcbr/Francais/EWCBR2000/Abstracts/ABST126.htm; Kocsis et al., Acta Pharm Hung 2002;72(1):49-61). Dabei ist wahrscheinlich der Wirkmechanismus von Tolperison etwas unterschiedlich zu dem von Lidocain. Es gibt außerdem Hinweise darauf, dass Tolperison die Natriumpermeabilität herabsetzt. Dieser Effekt könnte für die erregbarkeitsverringernde Wirkung von Tolperison verantwortlich sein und somit für die antispastische Wirkung, die in klinischen Beobachtungen dokumentiert werden konnte (Hinck and Koppenhofer, Gen Physiol Biophys 2001; 20:413-29). Zusätzlich konnte in Voltageclamp Experimenten an Schneckenneuronen gezeigt werden, dass Tolperison und seine Analoga spannungsabhängige Kalziumströme inhibieren (Novalies-Li et al., Eur J Pharmacol 1989; 168:299-305). Tolperisonanaloga wie Eperison und Silperison zeigten ein ähnliches Verhalten in elektrophysiologischen Experimenten. So konnte z.B. gezeigt werden, dass Silperison die Natriumpermeabilität verringert (During and Koppenhofer, Gen Physiol Biophys 2001; 20:157-73). Daraus kann geschlossen werden, dass diese Substanzen den spastischen Skelettmuskeltonus verringern könnten.

In klinischen Studien konnte weiterhin gezeigt werden, dass diese Substanzen schmerzhafte Spasmen lindern können, die mit neurologischen oder rheumatoiden Erkrankungen einhergehen. Es wurde beschrieben, dass Tolperison wirksam in der Behandlung von Muskelspasmen eingesetzt wird (Pratzel et al., Pain 1996; 67:417-25). Einige Derivate des Tolperison z.B. Eperison zeigten ebenfalls Wirksamkeit bei der Behandlung von schmerzhaften Muskelspasmen (Bose, Methods Find Exp Clin Pharmacol 1999; 21:209-13). Unter bestimmten pathologischen Bedingungen befinden sich Neuronen in einem Zustand fortwährender Depolarisation, so dass ihre Natriumkanäle sensitiver auf die inhibitorische Wirkung bestimmter Substanzen reagieren. Dadurch ist die Möglichkeit gegeben, Muskelspasmen und Schmerz mit einem günstigen Nebenwirkungsprofil zu lindern. Neuere Daten weisen darauf hin, dass Tolperison und seine Analoga selektiv inhibitorisch auf spannungsabhängige Natriumkanäle wirken. Dieser Mechanismus könnte für ihre Spinalreflex supprimierende und Muskel relaxierende Wirkung verantwortlich sein. Zusätzlich könnte diese Eigenschaft den schmerzlindernden Effekt bewirken, der aufgrund von den beobachteten geringen Unterschieden frei von Nebenwirkungen sein könnte im Gegensatz zu Lidocain.

Eine weitere Klasse muskelrelaxierender Substanzen sind die Kaliumkanalöffner. Dazu gehört beispielsweise Flupirtin aus einer Klasse von Triaminopyridinen, das als nichtopioides Analgetikum mit muskelrelaxierenden Eigenschaften eingesetzt wird. Es konnte gezeigt werden, dass Flupirtin den Skelettmuskeltonus senkt, wenn es in Dosen eingesetzt wird, die denen der antinociceptiven Wirkung vergleichbar sind (Nickel et al., Arzn Forsch/Drug Res 1990a; 40:909-11).
Da Diazepam und andere Benzodiazepine häufig als Muskelrelaxantien eingesetzt werden war es naheliegend, die pharmakodynamischen Eigenschaften von Flupirtin mit denen der Benzodiazepine zu vergleichen. In Rezeptorbindungsstudien wurde bis zu einer Konzentration von 10 µmol/l keine Affinität für spezifisches [³H]flunitrazepam nachgewiesen (Nickel et al., Arzn Forsch/Drug Res 1990b; 40:905-908). Im Bezug auf die Änderungen des EEG konnten deutliche Unterschiede in den Profilen nachgewiesen werden, die durch Flupirtin bzw. Benzodiazepine induziert werden (Nickel, Postgrad Med J 1987; 63:19-28). Elektrophysiologische Untersuchungen zeigten, dass Flupirtin die GABAerge Weiterleitung durch Potenzierung der GABA Wirkung beeinflußt (Weiser et al., Arch Pharmacol 1992; 346(Suppl.):R22). Daten aus *in vitro* und *in vivo* Analysen lassen vermuten, dass sich Flupirtin wie ein funktioneller N-Methyl-D-Aspartat (NMDA) - Antagonist verhält. Daraus könnte geschlossen werden, dass dieser Mechanismus an der muskelrelaxierenden Wirkung von Flupirtin beteiligt sein könnte (Schwarz et al., Neuroreport 1994; 5:1981-4). Neuere Untersuchungen zeigen auf, dass Flupirtin spannungsunabhängige Kaliumkanäle aktiviert (Komhuber et al., J Neural Transm 1999; 106:857-67). Dieser Kaliumkanal-öffnende Effekt von Flupirtin könnte für seine analgetische und Skelettmuskel-relaxierende Wirkung verantwortlich sein.

Der beschriebene Stand der Technik zeigt deutlich, dass es zwar eine Reihe an Substanzen gibt, die zur Behandlung von Schmerzzuständen mit erhöhtem Muskeltonus eingesetzt werden. So wird beispielsweise eine Kombination aus Flupirtin und Diclofenac zur Behandlung von Schmerzzuständen bei Arthrose und Arthritis eingesetzt (EP-A-0 189 788). Es sind jedoch in häufig Limitierungen gesetzt durch unerwünschte Nebenwirkungen. So zeigt beispielsweise Flupirtin bei höherer Dosierung neurotoxische Effekte wie Schläfrigkeit, Koordinationsstörung. Tolperison zeigt keine schweren unerwünschten Nebeneffekte, seine Wirksamkeit und Wirkungsdauer bei der Muskelrelaxation sind jedoch nicht zufriedenstellend, möglicherweise bedingt durch die relativ geringe Bioverfügbarkeit und die kurze Halbwertszeit im Menschen (Ito et al., Arch Int Pharmacodyn Ther 1985; 275:105-22, Matsunaga et al., Jpn J Pharmacol 1997; 73:215-20).

Aufhabe dieser Erfindung ist daher die Bereitstellung eines Arzneimittels zur Behandlung von Schmerzzuständen, die mit erhöhtem Muskeitonus einhergehen, das bei vergleichbaren Wirkung geringere Nebenwirkungen zeigt oder das bei gleicher Dosis eine erhöhte Wirksamkeit aufweist.

Dies konnte erfindungsgemäß durch die neue Kombination des Kaliumkanalöffners Flupirtin und eines Natriumkanalinhibitors aus der Gruppe enthaltend Tolperison, oder seine Analoga Eperison oder Silperison, oder deren pharmazeutisch verwendbare Salze bewirkt werden.
Es konnte gezeigt werden, dass durch die Kombination aus Natriumkanal inhibierenden oder beeinflussenden Wirkstoffen und Kaliumkanalöffnem die muskelrelaxierende Wirkung erhöht wird.

Als Na⁺-Kanal inhibierende oder beeinflussende Substanzen werden Tolperison oder seine Analoga Eperison und Silperison, sowie deren pharmazeutisch verwendbare Salze eingesetzt Als Kaliumkanalöfner wird Flupirtin aufgeführt

Durch die erfindungsgemäße Kombination wird die Behandlung von Schmerzzuständen, die mit erhöhtem Muskeltonus einhergehen, effektiver und sicherer. Die Kombination aus Na-Kanal inhibierenden oder beeinflussenden Substanzen und Flupirtin führt zu einer erhöhten therapeutischen Wirkung oder verbesserten Verträglichkeit. Beispielsweise konnte gezeigt werden, dass die Muskel relaxierende Wirkung von Flupirtin durch Na Kanal inhibierende oder beeinflussende Wirkstoffe wie Tolperison verstärkt werden kann und umgekehrt. Überraschend und für den Fachmann unerwartet ist jedoch insbesondere der Effekt, dass die Skelettmuskel relaxierende Wirkung von Flupirtin durch Tolperison überadditiv verstärkt wird und umgekehrt. Im Gegensatz dazu wird die Neurotoxizität von Flupirtin durch Tolperison nicht verstärkt.

Die Kombination beider Substanzen kann eingesetzt werden zur Behandlung von Schmerzzuständen bei Erkrankungen der Skelettmuskulatur, die mit Hypermyotonie und eingeschränkter Beweglichkeit einhergehen, insbesondere solche, die hervorgerufen werden durch Verletzungen des Rückenmarks, Osteoporose, Arthritis und Versteifung/krampfartigen Zuständen. Sie ist außerdem wirksam bei Schmerzzuständen folgender Genese: lumboischialen Schmerzen, Neurolathyrismus, Arthritis, Erkrankungen des peripheren Kreislaufsystems, klimakterischen Muskel- und Gefäßbeschwerden, Trismus, myogenen Kopfschmerzen, rheumatischen Erkrankungen, die mit Muskelhypertonie einhergehen, Spasmen, Schmerz, Entzündungssymptomen und eingeschränkter Beweglichkeit, multipler Sklerose und in der postoperativen Behandlung traumatischer Patienten, sowie zur Behandlung von unteren spastischen Paraparesesyndrom: unterem Paraspasmus, transversaler Myelitis, multipler Sklerose, vererbbarer inferiorer spastischer Paraplegie (Stuempel paraplegia), Störungen der spinalen Blutzirkulation, cerebraler Lähmung mit unterer spastischer Parese, Tetraparese bei zervikaler Myelopathie, vertebraler Dysplasie, Spannungskopfschmerz und zervikaler Brachialgie.

### Pharmakologische Beispiele

### 1: Muskelrelaxierende Wirkung auf die durch Reserpin induzierte Muskelrigität in Ratten

### Ergebnisse

Sowohl Flupirtin als auch Tolperison verringern die Reserpin induzierte Skelettmuskelstarre dosisabhängig in wachen Ratten Die intraperitoneale (i.p.) ED₅₀ für Flupirtin lag bei 6,45 mg/kg. Der ED₅₀ Wert für Tolperison war 32,4 mg/kg i.p.
Die Ergebnisse der Tabellen 1 und 2 zeigen deutlich, dass überraschend der Skelettmuskel relaxierende Effekt von Flupirtin durch Tolperison überadditiv verstärkt wird und umgekehrt.

**Tabelle 1. Wirkung von intraperitoneal verabreichtem Flupirtin in Kombination mit Tolperison auf Reserpin induzierte Skelettmuskelstarre in wachen Ratten**

| Behandlung | | Muskelrelaxation (%) | |
|---|---|---|---|
| | | berechnet | gemessen |
| Flupirtin 5 mg/kg | + Tolperison 12,5 mg/kg | 52,2 | 71,1* |
| Flupirtin 5 mg/kg | + Tolperison 25 mg/kg | 75,4 | 90.7* |
| Flupirtin 5 mg/kg | + Tolperison 50 mg/kg | 121,0 | 163.2* |

**Tabelle 2. Wirkung von intraperitoneal verabreichtem Tolperison in Kombination mit Flupirtin auf Reserpin induzierte Skelettmuskelstarre in wachen Ratten.**

| Behandlung | | Muskelrelaxation (%) | |
|---|---|---|---|
| | | berechnet | gemessen |
| Tolperison 25 mg/kg | + Flupirtin 1 mg/kg | 44.7 | 60.2* |
| Tolperison 25 mg/kg | + Flupirtin 3 mg/kg | 60.0 | 81.4* |
| Tolperison 25 mg/kg | + Flupirtin 5 mg/kg | 75.4 | 92.1* |

### Versuchsbeschreibung

Männliche Sprague-Dawley Ratten mit einem Gewicht von 200 - 220 g wurden in zweier Gruppen unter Standardbedingungen (Temperatur 22°C, Feuchte 40-60%) ohne Nahrungs- und Wasserbeschränkung gehalten. Beleuchtung war von 6 - 18 Uhr vorhanden. Die Experimente wurden vom örtlichen Tiergesundheitskomitee bewilligt, das für den Schutz und ordentlichen Einsatz von Versuchstieren verantwortlich ist.

Der experimentelle Ansatz wurde im Detail bereits beschrieben (Nickel et al., Arzn Forsch/Drug Res 1997; 47:1081-6). Kurz dargestellt wurde die Muskelstarre am Skelettmuskel gemessen, indem nacheinander der Widerstand der Flexor und Extensor Muskeln gemessen wurde, die beim Strecken und Beugen des Fußes im Gelenk entgegengesetzt wirken. Die Druckunterschiede, die durch die Bewegung des Fußes erzeugt wurden, wurden kontinuierlich aufgezeichnet. Die Signale wurden anhand eines PC-Programmes ausgewertet, das die Widerstandswerte von Flexor und Extensor am Fuß über Zeiträume von 10 min berechnet hat.

Die Wirkstoffe wurden täglich frisch angesetzt und 1 6 h nach der Reserpininjektion (2 mg/kg, intraperitoneal) simultan i.p. in verschiedenen Dosen verabreicht.

Die statistische Analyse der Unterschiede zwischen den kalkulierten und gemessenen Werten wurde durch one-way ANOVA durchgeführt. Sterne (*) bezeichnen den signifikanten Level p<0,01.

### 2: Untersuchungen des Skelettmuskeltonus von Mäusen im sogenannten "inclined screen test" (Schräggitter-Test)

### Ergebnisse

Die überraschenden Ergebnisse aus Beispiel 1 konnten überzeugend in einem Experiment mit Mäusen verifiziert werden.

Sowohl Flupirtin als auch Tolperison verringern den Skelettmuskeltonus dosisabhängig in wachen Mäusen und geben so Aufschluß über deren muskelrelaxierende Wirkung. Die intraperitoneale (i.p.) ED₅₀ für Flupirtin liegt bei 10.8 mg/kg. Der ED₅₀ Wert für Tolperison ist 51.0 mg/kg i.p..

Die Ergebnisse der Tabellen 3 und 4 zeigen deutlich, dass, bei simultaner i.p. Verabreichung verschiedener Dosen von Flupirtin und Tolperison, der Skelettmuskel relaxierende Effekt von Flupirtin durch Tolperison überadditiv verstärkt wird und umgekehrt.

**Tabelle 3. Wirkung von intraperitoneal verabreichtem Flupirtin in Kombination mit Tolperison auf den Skelettmuskeltonus von wachen Mäusen.**

| Behandlung | | Anzahl Tiere von der Schräge fallend in % | |
|---|---|---|---|
| | | berechnet | gemessen |
| Flupirtin 1 mg/kg | + Tolperison 12.5 mg/kg | 14 | 54* |
| Flupirtin 1 mg/kg | + Tolperison 25 mg/kg | 28 | 62* |
| Flupirtin 1 mg/kg | + Tolperison 50 mg/kg | 54 | 75* |

**Tabelle 4. Wirkung von intraperitoneal verabreichtem Tolperison in Kombination mit Flupirtin auf den Skelettmuskeltonus von wachen Mäusen.**

| Behandlung | | Anzahl Tiere von der Schräge fallend in % | |
|---|---|---|---|
| | | berechnet | gemessen |
| Tolperison 25 mg/kg | + Flupirtin 1 mg/kg | 28 | 50* |
| Tolperison 25 mg/kg | + Flupirtin 3 mg/kg | 37 | 60* |
| Tolperison 25 mg/kg | + Flupirtin 5 mg/kg | 46 | 70* |

### Versuchsbeschreibung

NMRI Mäuse mit einem Gewicht von 22 - 24 g wurden in vierer Gruppen unter Standardbedingungen (Temperatur 22°C, Feuchte 40-60%) ohne Nahrungs- und Wasserbeschränkung gehalten. Beleuchtung war von 6 - 18 Uhr vorhanden. Alle Experimente wurden vom örtlichen Tiergesundheitskomitee bewilligt, das für den Schutz und ordentlichen Einsatz von Versuchstieren verantwortlich ist.

Als pharmakologisches Modell, das Vorhersagen über die muskelrelaxierenden Eigenschaften ermöglicht, wurde der sogenannte "30 degrees inclined screen test" ("Schräggitter-Test") eingesetzt (Simiand et al., Arch Int Pharmacodyn Ther 1989; 297:272-85). Das Schräggitter besteht aus einem Holzrahmen mit einem Maschendrahtgitter, das in einem beliebigen Winkel geneigt werden kann (hier: 80°). Der untere Teil des Gitters befindet sich 15 cm über dem Tisch. Die Tiere werden auf das Schräggitter gesetzt, und ihre Fähigkeit, sich auf dem schrägen Gitter zu halten, wird über einen Zeitraum von 30 s beobachtet. Die Anzahl der Tiere, die vom Gitter fallen, wird gezählt und ihr Anteil an der Gesamtzahl jeder Gruppe berechnet.

Die Wirkstoffe wurden täglich frisch angesetzt und 1 h vor Beginn der Experimente simultan i.p. in verschiedenen Dosen zur Analyse des Skelettmuskeltonus verabreicht.

Die statistische Analyse der Unterschiede zwischen den kalkulierten und gemessenen Werten wurde durch one-way ANOVA durchgeführt. Sterne (*) bezeichnen den signifikanten Level p<0.01.

### 3: Mögliche neurotoxische Wirkungen der Substanzen, gemessen im rotating rod (Drehstab) test an Ratten

### Ergebnisse

Zentral wirkende Substanzen können neurotoxische Nebeneffekte haben, durch die ihr therapeutischer Einsatz eingeschränkt werden könnte. Die Ergebnisse der Tabellen 5 und 6 zeigen deutlich, dass die motorische Koordination durch die Kombination von Flupirtin und Tolperison additiv beeinflußt wird. Ein überadditiver Effekt kann nicht beobachtet werden, d.h. die Kombination Flupirtin + Tolperison führt nicht zur Steigerung unerwünschter zentralnervöser Wirkungen.

**Tabelle 5. Wirkung intraperitoneal verabreichten Flupirtins in Kombination mit Tolperison auf die motorische Koordination von Ratten mit Hilfe des "rotating rod".**

| Behandlung | | Anzahl Tiere von der Schräge fallend in % | |
|---|---|---|---|
| | | berechnet | gemessen |
| Flupirtin 1 mg/kg | + Tolperison 12.5 mg/kg | 38 | 42 |
| Flupirtin 1 mg/kg | + Tolperison 25 mg/kg | 50 | 49 |
| Flupirtin 1 mg/kg | + Tolperison 50 mg/kg | 70 | 67 |

**Tabelle 6. Wirkung intraperitoneal verabreichten Tolperisons in Kombination mit Flupirtin auf die motorische Koordination von Ratten mit Hilfe des "rotating rod".**

| Behandlung | | Anzahl Tiere von der Schräge fallend in % | |
|---|---|---|---|
| | | berechnet | gemessen |
| Tolperison 25 mg/kg | + Flupirtin 1 mg/kg | 49 | 50 |
| Tolperison 25 mg/kg | + Flupirtin 3 mg/kg | 57 | 50 |
| Tolperison 25 mg/kg | + Flupirtin 5 mg/kg | 66 | 67 |

### Versuchsbeschreibung

Männliche Sprague-Dawley Ratten mit einem Gewicht von 200 - 220 g wurden in zweier Gruppen unter Standardbedingungen (Temperatur 22°C, Feuchte 40-60%) ohne Nahrungs- und Wasserbeschränkung gehalten. Beleuchtung war von 6 - 18 Uhr vorhanden. Die Experimente wurden vom örtlichen Tiergesundheitskomitee bewilligt, das für den Schutz und ordentlichen Einsatz von Versuchstieren verantwortlich ist.

Die motorische Koordination und Balance der Tiere wurde im sogenannten "rotating rod test" (Drehstab) (Jones and Roberts, J Pharm Pharmacol 1968; 20:302-304) analysiert. Die Tiere werden auf einen rotierenden Stab gesetzt (Durchmesser 10 cm; Länge 60 cm; 5 rpm), und nach einem Zeitraum von 2 Minuten wird die auf dem Stab verbliebene Anzahl der Tiere gezählt. Die Wirkstoffe wurden täglich frisch angesetzt und 30 min vor Beginn der Experimente simultan intraperitoneal in verschiedenen Dosen verabreicht.

Die beschriebenen Experimente zeigen eindeutig die Wirkungen der Kombination Flupirtin/Tolperison. Aus den vergleichbaren Wirkmechanismen der Kaliumkanlöffner und Natriumkanal inhibierenden oder beeinflussenden Substanzen kann die gleiche positive Wirkung anderer Kombinationen von Stoffen dieser Substanzklassen hergeleitet werden.

Die Kombinationen aus Na⁺-Kanal inhibierenden oder beeinflussenden Wirkstoffen aus der Gruppe enthaltend Tolperison oder seine Analoga Eperison oder Silperison, oder deren pharmazeutisch verwendbare Salze und Flupirtin und ihrer pharmazeutisch verwendbaren Salze, können in allen oralen, enteralen, rectalen, lingualen, intravenösen, intramuskulären, intraperitonealen, transdermalen, subcutanen oder intracutanen Darreichungsfomen verabreicht werden. Bevorzugte orale Darreichungsformen sind beispielsweise Tabletten, Filmtabletten, Dragees, Hartkapseln, Weichkapseln, Kautabletten, Lutschtabletten, Sirup, Präparationen mit kontrollierter Freisetzung (z.B. dual-Formulierung, Retardformulierung), Pellets, Kautabletten oder lösliche Granulate. Als weitere Darreichungsformen kommen beispielsweise in Frage: Injektionslösungen, Suspensionen, Suppositorien, Cremes, Salben, Gele, transdermale Applikationsformen, sub- oder intracutane Implantate.

Die Substanzen können gleichzeitig, nacheinander oder in einer fixen Kombination verabreicht werden. Sie können zusammen in einer Darreichungsform oder in zwei Darreichungsformen, die gleich oder verschieden sein können, verabreicht werden. Sie können gleichzeitig (simultan) oder nacheinander verabreicht werden, entweder kurz nacheinander oder mit größeren Zeitabständen, z.B. Flupirtin abends und Tolperison am Morgen.

Die Wirkstoffe können zwischen 1 und 8 mal täglich verabreicht werden, in ausreichender Menge um die gewünschte Wirkung zu erzielen. Vorzugsweise werden die Wirkstoffe ein bis viermal täglich verabreicht.

Die tägliche Dosis sollte in Übereinstimmung mit der zugelassenen Menge der jeweils in der Kombination eingesetzten Substanzen liegen. Dies ist für die bevorzugte Kombination beispielsweise zwischen 150 und 450 mg/Tag Tolperison beim Erwachsenen, Flupirtin bei 100 - 800 mg/Tag, bevorzugt zwischen 200 und 400 mg/Tag.

## Patentansprüche

1. Verwendung von Flupirtin in Kombination mit Tolperison oder seinen Analoga Eperison oder Silperison, oder deren pharmazeutisch verwendbaren Salzen zur Herstellung eines Arzneimittels zur Behandlung von Schmerzzuständen, die mit erhöhtem Muskeltonus einhergehen.

2. Verwendung von Flupirtin in Kombination mit Tolperison oder seinen Analoga Eperison oder Silperison, oder deren pharmazeutisch verwendbaren Salzen gemäß Anspruch 1, **dadurch gekennzeichnet, dass** es sich bei den Schmerzzuständen um Neuralgien handelt.

3. Verwendung von Flupirtin in Kombination mit Tolperison oder seinen Analoga Eperison oder Silperison, oder deren pharmazeutisch verwendbaren Salzen gemäß Anspruch 1, **dadurch gekennzeichnet, dass** es sich bei den Schmerzzuständen um Arthritis oder Arthrose handelt.

4. Verwendung von Flupirtin in Kombination mit Tolperison oder seinen Analoga Eperison oder Silperison, oder deren pharmazeutisch verwendbaren Salzen gemäß Anspruch 1, **dadurch gekennzeichnet, dass** es sich bei den Schmerzzuständen um chronischen oder episodischen Spannungskopfschmerz handelt.

5. Verwendung von Flupirtin in Kombination mit Tolperison oder seinen Analoga Eperison oder Silperison, oder deren pharmazeutisch verwendbaren Salzen gemäß Anspruch 1, **dadurch gekennzeichnet, dass** es sich bei den Schmerzzuständen um unteres spastisches Paraparesesyndrom (z.B unterer Paraspasmus, transversale Myelitis, multiple Sklerose, Vererbbare inferiore spastische Paraplegie (Stuempel paraplegia), Störungen der spinalen Blutzirkulation, cerebrale Lähmung mit unterer spastischer Parese) handelt.

6. Verwendung von Flupirtin in Kombination mit Tolperison oder seinen Analoga Eperison oder Silperison, oder deren pharmazeutisch verwendbaren Salzen gemäß Anspruch 1, **dadurch gekennzeichnet, dass** es sich bei den Schmerzzuständen um Tetraparese bei zervikaler Myelopathie, zervikaler Brachialgie oder vertebraler Dysplasie handelt.

7. Verwendung von Flupirtin in Kombination mit Tolperison oder seinen Analoga Eperison oder Silperison, oder deren pharmazeutisch verwendbaren Salzen gemäß Anspruch 1, **dadurch gekennzeichnet, dass** es sich bei den Schmerzzuständen um die Parkinson Krankheit handelt.

8. Verwendung von Flupirtin in Kombination mit Tolperison oder seinen Analoga Eperison oder Silperison, oder deren pharmazeutisch verwendbaren Salzen gemäß Anspruch 1, **dadurch gekennzeichnet, dass** das Medikament zur oralen, rektalen, intravenösen, transdermalen, sub- oder intracutanen Verabreichung geeignet ist.

## Claims

1. Use of flupirtine in combination with tolperisone or its analogues eperisone or silperisone, or their pharmaceutically utilizable salts, for producing a medicament for treating pains which are accompanied by an increase in muscle tone.

2. Use of flupirtine in combination with tolperisone or its analogues eperisone or silperisone, or their pharmaceutically utilizable salts, according to Claim 1, **characterized in that** the pains are neuralgias.

3. Use of flupirtine in combination with tolperisone or its analogues eperisone or silperisone, or their pharmaceutically utilizable salts, according to Claim 1, **characterized in that** the pains are arthritis or arthrosis.

4. Use of flupirtine in combination with tolperisone or its analogues eperisone or silperisone, or their pharmaceutically utilizable salts, according to Claim 1, **characterized in that** the pains are chronic or episodic tension headache.

5. Use of flupirtine in combination with tolperisone or its analogues eperisone or silperisone, or their pharmaceutically utilizable salts, according to Claim 1, **characterized in that** the pains are lower spastic paraparesis syndrome (e.g. lower paraspasm, transverse myelitis, multiple sclerosis, heritable inferior spastic paraplegia (Stuempel paraplegia), disturbances of the spinal blood circulation and cerebral paralysis involving lower spastic paresis).

6. Use of flupirtine in combination with tolperisone or its analogues eperisone or silperisone, or their pharmaceutically utilizable salts, according to Claim 1, **characterized in that** the pains are tetraparesis in connection with cervical myelopathy, cervical brachialgia or vertebral dysplasia.

7. Use of flupirtine in combination with tolperisone or its analogues eperisone or silperisone, or their pharmaceutically utilizable salts, according to Claim 1, **characterized in that** the pains are Parkinson's disease.

8. Use of flupirtine in combination with tolperisone or its analogues eperisone or silperisone, or their pharmaceutically utilizable salts, according to Claim 1, **characterized in that** the medicament is suitable for oral, rectal, intravenous, transdermal or subcutaneous or intracutaneous administration.

## Revendications

1. Utilisation de flupirtine en combinaison avec de la tolpérisone ou ses analogues épérisone ou silpérisone, ou ses sels pharmaceutiquement utilisables pour la préparation d'un médicament destiné au traitement d'états de douleur, qui vont de pair avec un tonus musculaire augmenté.

2. Utilisation de flupirtine en combinaison avec de la tolpérisone ou ses analogues épérisone ou silpérisone, ou ses sels pharmaceutiquement utilisables selon la revendication 1, **caractérisée en ce qu'**il s'agit, pour les états de douleur, de névralgies.

3. Utilisation de flupirtine en combinaison avec de la tolpérisone ou ses analogues épérisone ou silpérisone, ou ses sels pharmaceutiquement utilisables selon la revendication 1, **caractérisée en ce qu'**il s'agit, pour les états de douleur, d'arthrite ou d'arthrose.

4. Utilisation de flupirtine en combinaison avec de la tolpérisone ou ses analogues épérisone ou silpérisone, ou ses sels pharmaceutiquement utilisables selon la revendication 1, **caractérisée en ce qu'**il s'agit, pour les états de douleur, de céphalées de tension chroniques ou épisodiques.

5. Utilisation de flupirtine en combinaison avec de la tolpérisone ou ses analogues épérisone ou silpérisone, ou ses sels pharmaceutiquement utilisables selon la revendication 1, **caractérisée en ce qu'**il s'agit, pour les états de douleur, du syndrome de la paraparésie spastique (par exemple le paraspasme des membres inférieurs, la myélite transverse, la sclérose en plaques, la paraplégie spastique héréditaire des membres inférieurs (paraplégie de Stümpel), les troubles de la circulation sanguine spinale, la paralysie cérébrale avec une parésie spastique des membres inférieurs).

6. Utilisation de flupirtine en combinaison avec de la tolpérisone ou ses analogues épérisone ou silpérisone, ou ses sels pharmaceutiquement utilisables selon la revendication 1, **caractérisée en ce qu'**il s'agit, pour les états de douleur, d'une tétraparésie lors d'une myélopathie cervicale, d'une brachialgie cervicale ou d'une dysplasie vertébrale.

7. Utilisation de flupirtine en combinaison avec de la tolpérisone ou ses analogues épérisone ou silpérisone, ou ses sels pharmaceutiquement utilisables selon la revendication 1, **caractérisée en ce qu'**il s'.agit, pour les états de douleur, de la maladie de Parkinson.

8. Utilisation de flupirtine en combinaison avec de la tolpérisone ou ses analogues épérisone ou silpérisone, ou ses sels pharmaceutiquement utilisables selon la revendication 1, **caractérisée en ce que** le médicament convient pour une administration par voie orale, rectale, intraveineuse, transdermique, sous-cutanée ou intracutanée.
